Europäisches Patentamt

European Patent Office

Office européen des brevets

(19)

(11) Publication number: **0 158 108 B1**

## EUROPEAN PATENT SPECIFICATION

(12)

(45) Date of publication of patent specification: **14.10.92**

(21) Application number: **85102421.6**

(22) Date of filing: **04.03.85**

(51) Int. Cl.⁵: **A61K 7/48**, A61K 7/50, A61K 7/08

The file contains technical information submitted after the application was filed and not included in this specification

(54) **Detergent composition.**

(30) Priority: **05.03.84 IT 1989884**
**13.02.85 IT 1949085**

(43) Date of publication of application:
**16.10.85 Bulletin 85/42**

(45) Publication of the grant of the patent:
**14.10.92 Bulletin 92/42**

(84) Designated Contracting States:
**AT BE CH DE FR GB IT LI LU NL SE**

(56) References cited:
DE-A- 2 243 281    FR-A- 1 086 004
FR-A- 1 098 828    FR-A- 1 199 691
FR-A- 1 526 808    FR-A- 2 252 403
FR-A- 2 496 458    FR-A- 2 515 034
GB-A- 460 839

**H. JANISTYN, "Handbuch der Kosmetika und Riechstoffe", vol. 3, 2nd edition, Die Körperpflegemittel, 1973, Dr. Alfred Hüthig Verlag, Heidelberg (DE)***

(73) Proprietor: **TONFER INC.**
**22 Crestmont Road**
**Montclair New Jersey 07042(US)**

(72) Inventor: **Ferro, Antonio**
**Via Maraini 7**
**Lugano(CH)**

(74) Representative: **Perani, Aurelio et al**
**c/o JACOBACCI-CASETTA & PERANI S.p.A 7,**
**Via Visconti di Modrone**
**I-20122 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a detergent composition for the hair and skin and, more specifically, to a detergent composition which is essentially non irritating and capable of maintaining the skin in a smoother and tender condition.

The mechanism of skin cleaning is well known and has been largely studied: this problem has been always faced by using chemical compounds having specific detergent action.

Since a detergent action, more or less strong but nevertheless specific, is accompanied by not negligible side effects, such as for instance some dryness of the skin, combined with phenomena like chaps, roughness, and blushing, and since these problems have not only an aesthetic consequence but also involve more generally the natural defences of the skin, a great number of attempts have been proposed and carried out throughout the years, essentially consisting in the provision of complex formulations; the latter, besides the component having specific detergent action contain other ingredients, such as for example emollient substances capable or reducing the skin roughness as induced by the cleaning. As a typical example, the lanoline and other animal, vegetal or mineral oils can be cited, which in definitely modest percentages are present in the detergent compositions, both solid and liquid.

Another common expedient is that of adjusting the pH of the cleaning composition by bringing it towards the neutrality.

The use of oily mixtures for the care of the skin and of hair, both for detergent and for cosmetic purposes, has been already proposed in the past.

According to the prior art these compositions were applied by rubbing the skin and/or the hair and then removed.

Possibly the composition was added with a surface active agent or emulsifying agent, in order to promote the removal of the composition by rinsing with water.

Consequently according to the prior art it was taught to apply these compositions to the skin and/or the hair by rubbing, maintaining the composition into contact with the skin and/or the hair for some time, for instance some minutes or more, and thereafter rinsing with water, taking advantage of the fact that the presence of an emulsifying agent causes an emulsion to be formed by which the composition is more easily removed.

In the pediatric field oily compositions have been and are used, with which the body is rubbed in the areas covered by absorbing napkins, in order to counterweight the macerating effect of the body liquids, and of faeces. The explanation resides also in that the baby skin is too delicate to stand the detergent action even of very mild soaps.

In this connection it is worth reviewing the properties and main features of the lipidic film or sebum coating the skin. It is a hydrophilic, homogeneous, finely emulsified layer having important protecting functions.

This lipidic film is generated, in varying amounts and composition, throughout the life of the human being both directly onto the surface of the skin and by suitable sebaceous glands.

Some times, the sebum production is in excess with respect to the need of the skin (seborrhea) or in shortcoming (alipic skin) and in both cases disorders, anomalies, alterations and cutaneous defects are induced; thus the importance of the sebum and of its biological role can be appreciated.

Sebum as a matter of fact protects the skin from the atmospherical pollutants, from cutaneous steeping, from dehydration and from abrupt temperature variations. Moreover it acts as a surface shield against chemical aggressive substances, hindering their contact with the skin and their absoprtion. It acts also as a carrier for the precursors of vitamin D and is endowed with a relevant anti-infective activity against several pathogenic micro-organisms.

Lastly, sebum has a lubricating, emollient and lenitive effect, thus inducing skin softness and turgidity and preventing an excessive evaporation of the surface water. Sebum is a semisolid lipidic mixture, consisting of triglycerides, free fatty acids, waxes, sterols and related esters,alcohols and hydrocarbons (among which a typical example is squalene) together with appreciable amounts of vitamins (mainly provitamin D and vitamins A and E).

Sebum, owing to the presence of cholesterol and of its esters, gives place to a water/oil emulsion, which although being capable of including relevant amounts of water, maintains its lipidic non hydrodispersible character, whereby it can not be removed from the skin with the help of water alone.

The skin cleaning means the removal of sebum, of waste and of dirt, but is not a physiological operation since it causes most of times the aforesaid lipidic protective layer to be removed too.

The skin cleaning is however a necessary operation since not only dirt and wastes are to be removed, but also a portion of the surface sebum layer, namely the older one as regards its formation, must be taken away. In fact sebum, some time after its generation, undergoes chemical changes, owing to both the oxygen of the air and to microorganisms normally present onto the skin, these changes making the sebum irritating for the skin.

Consequently, the need existed and exists of means capable of efficaciously removing from the skin wastes, dirt and the surface portion of the sebum, without fully removing the sebum layer, and without irritating the skin.

Under this point of view, apart from soaps and detergent compositons, affected by the afore-said drawbacks, also the so-called cleaning milks are not suitable, since their cleaning action is not sufficient, both as regards the removal of the surface part of the sebum, and as regards the removal of heavier make up or dirt.

The composition according to the present invention does fully solve the above mentioned problems and to this end consists in a mixture comprising:

a) a greater proportion of at least one oil selected among mineral, vegetal, animal and synthetic oils, and

b) an emulsifying mixture of at least two emulsifying agents, soluble in the oily component (a) one of which is a saccharose ester selected among saccharose mono- and di-laurate, mono- and di-palmitate and mono- and di-stearate.

As already indicated one of the essentiall component of the compositions of the present invention is an oil or a mixture of vegetal, mineral or animal oils.

As examples of oils useful in the present invention, the following can be cited.

For the mineral oils:

Vaseline oil, silicone oil.

For the vegetal oils:

almond oil, mais oil, olive oil, walnut oil, soya oil, grape-stone oil, sesame oil, peanut oil, coconut oil, avocado oil or sunflower oil.

For the animal oils:

Turtle oil, mink oil, marmot oil or lanoline oil.

Among the synthetic oils, those already known in the cosmetic field can be used, such as for instance isopropyl myristate, palmitate and stearate, 2-octyldodecanol, decyl oleate, derivatives of isostearic acid, diisopropyladipate or myristyllactate. As a total or partial substitution for the oil the use is foreseen of a possibly vegetal gelatine, and in that case the density of the detergent composition of the invention can also be adjusted.

The percent contents of oil or oil mixture in the subject composition varies between 80 and 98% by weight and is preferably of the order of 90%.

Another essential component of the compositions according to the invention consists of an emulsifying mixture soluble in the subject oils, selected among anionic, cationic, non ionic and amphoteric emulsifiers. Specific examples of emulsifiers useful in the compositions of the present invention comprise:

- fatty alcohol sulphates and derivatives;
- quaternary ammonium compounds and derivatives;
- derivatives of aminoacids, betain;
- fatty acids and alcohols condensed with ethylene oxide;
- polyethylene and polyoxypropylene derivatives.

As it is well known the emulsifying agents have no specific detergent action whereby as a matter of principle the combination of an oil and of an emulsifying substance should not have detergent action, but in fact the most surprising feature is that this combination acts as a soap when combined with water before being applied to the skin and is able to leave a lipidic layer onto the skin which means that only a portion of the sebum layer is removed.

The subject emulsifiers are in turn contained in the composition of the invention in a percentage by weight of between 2 and 20%.

According to a further feature of the invention a minor percentage of the emulsifiers can be substituted for by a conventional surface active agent.

In this connection it is however to be pointed out that, in comparison with the prevailing amount of the oily component, the small percentage of surface active agent can be certainly responsible for the detergent action.

According to the main feature of the present invention the presence is foreseen of a saccharose ester as one of the emulsifying agents, it has been found that they are endowed with a synergic action towards the other (one or more) emulsifying agents present in the composition, since the resulting emulsion is finer and is also softer and more flowable.

This fact has been demonstrated by tests carried out with a composition of the present invention, namely comprising an oily part and an emulsifying agent, by adding a saccharose ester selected among saccharose monolaurate, monopalmitate, monosterate and distearate, the saccharose ester being added in the amount of 0.5% by weight of the composition.

The control composition contained 72% of mineral oil, 25% of soya oil and 3% of polyoxythylenoleyl ether.

The results are reported in the following table as the transparency T of the resulting emulsion at 650 nm and for several grades of dilution.

|  | T at 650 nm | | |
|---|---|---|---|
|  | dilution 1:1000 | dilution 1:2500 | dilution 1:5000 |
| Control sample (without saccharose ester) | 48.8 | 78.9 | 81.6 |
| Sample with saccharose monolaurate | 30.4 | 62.5 | 75.0 |
| Sample with saccharose monopalmitate | 23.5 | 55.4 | 74.2 |
| Sample with saccharose monostearate | 26.3 | 58.8 | 76.0 |
| Sample with saccharose-distearate | 22.0 | 50.3 | 75.5 |

As it can be appreciated from the values of transmittance, in all cases, the addition of saccharose esters induces an increase of the turbidity of the oil/water emulsion which in turn gives place to a sensible increase of the milky consistency and thus to an increased detergent action.

As regards the concentration of the saccharose ester it varies between 0.05 and 2% by weight referred to the weight of the composition.

According to a further feature of the present invention the subject compositions are added with a powder clay of bentonite or montmorillonite type: in fact it has been found that by such an addition the thickness of the lipidic layer remaining on the skin after the rinsing with water can be controlled.

More particularly if the amount of the clay powder present in the composition is of between 0.5 and 1.5% by weight a more consistent thickness of the lipidic layer is obtained, which can be defined as making the skin overfatty, whereas if the content of clay powder is of between 1.5 and 10%, a creamy layer remains onto the skin.

Otherwise stated, if the content of clay powder present in the composition is in the above lower interval the skin feels to be soft and a little greasy, whereas if the content is in upper interval the skin appears as when a cream layer is applied.

According to a further feature of the invention the compositions of the present invention are also endowed with skin hydrating and restituting properties, as well as with a nutrient and revitalizing function.

For the hydrating and restituting action the composition can be added with hydrating agents, such as for instance glycerin, urea and aminoacids which are very poorly or not soluble and not dispersible in the oils, by which the skin dehydration, as caused by the removal of natural hydrating substances (as induced by the detergent action), is prevented.

As regards the subject aminoacids, valine, isovaline, leucine and isoleucine can be cited.

Their presence together with urea and glycerin gives place to the revitalizing action, whereas to get the nutrient effect a liposoluble vitamin (such as vitamin A, E or F) is added.

According to a further feature of the invention, the subject compositions are used as hair shampoo instead of the known shampooing compositions, the latter always causing the removal from the scalp and from the hair of an excess of protecting sebum, whereby a so-called reactive seborrhea is induced, which becomes worse if the washing frequency is increased.

The use of the compositions of the invention as hair shampoo prevents the reactive seborrhea from occurring both because the conventional detergents are absent and because a hydrolipidic sebum-like film remains in substitution for the removed natural sebum.

In this connection it is worth reviewing the properties of the compositions of the present invention:
- the skin pH remains unchanged;
- is fully tolerated by the skin and has no irritating action towards the eyes and the mucosae:
- does not cause allergic or photosensitizing effects;
- permits the removal from the skin of all types of residues, e.g. fatty make up;
- induces the removal of only the surface part of the sebum, namely that which possibly has undergone the greater alterations;
- no unpleasant residues remain on the skin;
- the epicutaneous lipidic layer is not strongly attacked, whereby the skin is safeguarded from blushing desquamation.

As an explanation of the favourable results of the compositions of the present invention, which however should not be considered as exhaustive or limiting, it is to be taken into account that the subject compositions, in the presence of water form a milky emulsion, most of which is of the type oil/water, and is thus washable, whereas a small proportion is of the opposite type (namely water/oil): the latter portion remains on the skin after the rinsing, thus giving place to the protective effect. In fact this layer substitutes the sebum portion which is removed, retaining its more important chemico-physical and biological properties, and being at the same time more stable towards the oxidizing agents and more resistant to the microorganism attack.

Some examples of formulation of the compositions of the invention are given hereinafter:

| | EXAMPLE 1 |
|---|---|
| glyceryl tricaprylate | 48.9% |
| Isopropyl myristate | 48.3% |
| Cholesterol condensed with ethylene oxide | 2% |
| Saccharose monopalmitate | 0.2% |
| antioxidant and perfume | enough |

| | EXAMPLE 2 |
|---|---|
| maïs oil | 46.8% |
| grape-stone oil | 46.9% |
| polyoxyethylen oleylether | 4% |
| glycerylmonostearate | 0.2% |
| sorbitan monolaurate | 2% |
| saccharose distearate | 0.1% |
| antioxidant and perfume | enough |

| | EXAMPLE 3 |
|---|---|
| mineral oil | 58.4% |
| sunflower oil | 25.05% |
| lanoline oil | 10% |
| polyethylenglycol stearate | 1.5% |
| polyoxyethylenoleylether | 3% |
| saccharose monolaurate | 0.05% |
| bentonite | 2% |
| antioxidant and perfume | enough |

| | EXAMPLE 4 |
|---|---|
| sweet almond oil | 44.8% |
| olive oil | 44.8% |
| polyoxyethylenoleylether | 5% |
| spermaceti | 0.2% |
| bees wax | 0.2% |
| cetyl alcohol condensed with ethylene oxide | 4% |
| cetyl lactate | 3% |
| saccharose monolaurate | 1% |
| bentonite | 3% |
| perfume | enough |

|  | EXAMPLE 5 |
|---|---|
| hydrogenated coconut oil | 36.5% |
| isopropylpalmitate | 36.5% |
| polyethylene-polypropylene glycol | 10% |
| glyceryl tricaprylate | 5% |
| stearyl alcohol condensed with ethylene oxide | 4% |
| cetyl lactate | 3% |
| saccharose monolaurate | 1% |
| perfume | enough |

|  | EXAMPLE 6 |
|---|---|
| diisopropyl adipate | 38.75% |
| isostearyl alcohol | 38.75% |
| decyl oleate | 10% |
| sorbitan monostearate | 1.5% |
| polyoxyethylen lauryl alcohol | 2% |
| polyoxyethylen oleyl alcohol | 4% |
| montmorillonite | 5% |
| antioxidant and perfume | enough |

|  | EXAMPLE 7 |
|---|---|
| 70% vaseline oil | 54.11% |
| 30% sesame oil | 23.19% |
| 2-octyl dodecanol | 10% |
| glycerin | 5% |
| urea | 0.5% |
| polyoxyethylenoleylether | 6% |
| saccharose monopalmitate | 0.2% |
| antioxidant and perfume | enough |

|  | EXAMPLE 8 |
|---|---|
| lanoline oil | 78% |
| mink oil | 5% |
| marmot oil | 5% |
| avocado oil | 5% |
| glycerin | 5% |
| urea | 1% |
| aminoacids with aliphatic chains having a content of carbon atoms more than 4 | 1% |
| saccharose monostearate | 0.2% |
| bentonite | 5% |
| ethoxylated lanoline | 2% |
| polyoxyethylen lauryl ether | 5% |
| antioxidant and perfume | enough |

EP 0 158 108 B1

| | EXAMPLE 9 |
|---|---|
| 50% isostearyl alcohol | 36.2% |
| 50% isopropyl miristate | 36.2% |
| corn germ oil | 10% |
| perhydrosqualene | 5% |
| sorbitan hexaoleate | 2% |
| polyoxyethylen sorbitan monolaurate | 3% |
| polyoxyethylen sorbitan monostearate | 3% |
| bentonite | 1% |
| saccharose monolaurate | 0.1% |
| glycerin | 3% |
| urea | 0.5% |
| antioxidant and perfume | enough |

| | EXAMPLE 10 |
|---|---|
| vaseline oil | 30% |
| isopropyl miristate | 32% |
| castor oil | 29% |
| polyoxyethylen lauryl alcohol | 8% |
| saccharose laurate | 1% |

| | EXAMPLE 11 |
|---|---|
| isostearyl isostearate | 36% |
| soya oil | 54% |
| sorbitan monopalmitate | 2% |
| polyethylen oleylstearate | 4% |
| sorbitan trioleate | 4% |

| | EXAMPLE 12 |
|---|---|
| lanoline oil | 10% |
| silicon oil | 5% |
| olive oil | 30% |
| soya oil | 38% |
| isopropyl adipate | 5% |
| 2-octyldodecanol | 5% |
| polyoxypropylene stearyl alcohol | 5% |
| polyoxypropylene stearyl alcohol | 4% |
| polyoxyethyelene sorbitan monooleate | 3 |

The above last three examples specifically relate to shampooing compositions.

**Claims**

1. Detergent composition containing one oil selected among animal, vegetal, mineral and synthetic oils, as well as their mixtures, and an emulsifying mixture comprising at least two emulsifying agents, selected among anionic, cationic, non ionic and amphoteric emulsifying agents, characterized in that said emulsifying mixture comprises as one of the at least two emulsifying agents a saccharose ester selected among saccharose mono- and di-laurate, mono- and di-palmitate and mono- and di-stearate.

7

**2.** Detergent composition according to claim 1, characterized in that said saccharose ester is present in an amount of between 0,05 and 2% by weight referred to the weight of the composition.

**3.** Detergent composition according to claim 1, characterized in that said oil or oil mixture is at least partially substituted by vegetal gelatine.

**4.** Detergent composition according to claim 1, characterized in that the content of said oil or oil mixture is of between 80 and 98% by weight of the composition.

**5.** Detergent composition according to claim 1, characterized in that said emulsifying mixture comprises an emulsifying agent selected among:
- fatty alcohol sulphates and derivates;
- derivatives of aminoacids and betain;
- fatty acids and alcohols condensed with ethylene oxide;
- polyethylene and polyoxypropylene derivatives.

**6.** Detergent composition according to claim 1, characterized in that the content of said emulsifying mixture is of between 2 and 20% by weight of the composition.

**7.** Detergent composition according to claim 1, characterized in that said mineral oil is selected among vaseline oil and silicone oil.

**8.** Detergent composition according to claim 1, characterized in that said vegetal oil is selected among almond oil, maïs oil, olive oil, walnut oil, soya oil, grape-stone oil, sesame oil, peanut oil, coconut oil, avocado oil, sunflower oil and corn oil.

**9.** Detergent composition according to claim 1, characterized in that said animal oil is selected among turtle oil, mink oil, marmot oil and lanoline oil.

**10.** Detergent composition according to claim 1, characterized in that it further contains a hydrating agent.

**11.** Detergent composition according to claim 10, characterized in that said hydrating agent is selected among glycerin, urea and aminoacids non dispersible and non or poorly soluble in water, both alone and in combination.

**12.** Detergent composition according to claim 11, characterized in that said aminoacids are selected among valine, isovaline, leucine and isoleucine.

**13.** Detergent composition according to claim 1, characterized by containing a vitamin selected from vitamin A, E and F.

**14.** Detergent composition according to claim 1, characterized by further containing clay in powder form.

**15.** Detergent composition according to claim 14, characterized in that said clay is selected among bentonite and montmorillonite clays.

**16.** Detergent composition according to claim 14, characterized in that said powder clay is present in an amount of 0.5 to 10% referred to the weight of the composition.

**17.** Detergent composition according to claim 16, characterized in that said powder clay is present in the amount of 0.5 to 1.5% by weight of the composition.

**18.** Detergent composition according to claim 16, characterized in that said powder clay is present in the amount of 1.5 to 10% by weight of the composition.

**19.** Detergent composition according to claim 1, characterized in that said synthetic oil is selected among isopropyl myristate, palmitate and stearate, 2-octyl dodecanol, decyl oleate, derivatives of isostearic acid, diisopropyl adipate and myristyl lactate.

**20.** Method for the skin and hair cleaning characterized in that a composition according to one of the preceding claims is admixed with water to form an emulsion and applied to the skin or hair, the emulsion being then removed by rinsing.

**21.** Use of a saccharose ester selected among saccharose mono- and di-laurate, mono- and di-palmitate and mono- and di-stearate in the manufacturing of a detergent composition useful for the skin and hair cleansing comprising one oil component selected among animal, vegetal, mineral and synthetic oils, as well as their mixtures,and an emulsifying mixture comprising at least two emulsifying agents selected among anionic, cationic, non ionic and amphoteric emulsifying agents.

**Patentansprüche**

**1.** Waschmittelzusammensetzung, die ein Öl, ausgewählt aus tierischen, pflanzlichen, Mineral- und synthetischen Ölen sowie deren Mischungen, und eine emulgierende Mischung enthält, die mindestens zwei Emulgiermittel, ausgewählt aus anionischen, kationischen, nicht-ionischen und amphoteren Emulgiermitteln, umfaßt, dadurch gekennzeichnet, daß diese emulgierende Mischung als eines der mindestens zwei Emulgiermittel einen Saccharose-Ester umfaßt, der ausgewählt wird unter Saccharose-mono- und -dilaurat, -mono- und -dipalmitat und -mono- und -distearat.

**2.** Waschmittelzusammensetzung nach Anspruch 1, dadurch gekennzeichnet, daß der Saccharose-Ester in einer Menge von zwischen 0,05 und 2 Gew.-% bezogen auf das Gewicht der Zusammensetzung vorhanden ist.

**3.** Waschmittelzusammensetzung nach Anspruch 1, dadurch gekennzeichnet, daß das Öl oder die Ölmischung mindestens teilweise durch pflanzliche Gelatine ersetzt ist.

**4.** Waschmittelzusammensetzung nach Anspruch 1, dadurch gekennzeichnet, daß der Gehalt des Öls oder der Ölmischung zwischen 80 und 98 Gew.-% der Zusammensetzung beträgt.

**5.** Waschmittelzusammensetzung nach Anspruch 1, dadurch gekennzeichnet, daß die emulgierende Mischung ein Emulgiermittel umfaßt, das ausgewählt wird unter:
Fettalkoholsulfaten und Derivaten;
Derivaten von Aminosäuren und Betain;
Fettsäuren und Alkoholen, die mit Ethylenoxid kondensiert sind;
Polyethylen- und Polyoxypropylen-Derivaten.

**6.** Waschmittalzusammensetzung nach Anspruch 1, dadurch gekennzeichnet, daß der Gehalt der emulgierenden Mischung zwischen 2 und 20 Gew.-% der Zusammensetzung beträgt.

**7.** Waschmittelzusammensetzung nach Anspruch 1, dadurch gekennzeichnet, daß das Mineralöl aus Vaselineöl und Silikonöl ausgewählt wird.

**8.** Waschmittelzusammensetzung nach Anspruch 1, dadurch gekennzeichnet, daß das pflanzliche Öl ausgewählt wird aus Mandelöl, Maisöl, Olivenöl, Walnußöl, Sojaöl, Weinkernöl, Sesamöl, Erdnußöl, Kokosnußöl, Avocadoöl, Sonnenblumenöl und Maiskeimöl.

**9.** Waschmittelzusammensetzung nach Anspruch 1, dadurch gekennzeichnet, daß das tierische Öl ausgewählt wird aus Schildkrötenöl, Nerzöl, Murmeltieröl und Lanolinöl.

**10.** Waschmittelzusammensetzung nach Anspruch 1, dadurch gekennzeichnet, daß sie ferner ein hydratisierendes Mittel enthält.

**11.** Waschmittelzusammensetzung nach Anspruch 10, dadurch gekennzeichnet, daß das hydratisierende Mittel ausgewählt wird aus Glycerin, Harnstoff und Aminosäuren, die in Wasser nicht dispergierbar und nicht oder wenig löslich sind, sowohl einzeln als auch in Kombination;

**12.** Waschmittelzusammensetzung nach Anspruch 11, dadurch gekennzeichnet, daß die Aminosäuren aus Valin, Isovalin, Leucin und Isoleucin ausgewählt werden.

EP 0 158 108 B1

**13.** Waschmittelzusammensetzung nach Anspruch 1, dadurch gekennzeichnet, daß sie ein Vitamin enthält, das ausgewählt wird aus Vitamin A, E und F.

**14.** Waschmittelzusammensetzung nach Anspruch 1, dadurch gekennzeichnet, daß sie ferner Ton in Pulverform enthält.

**15.** Waschmittelzusammensetzung nach Anspruch 14, dadurch gekennzeichnet, daß dieser Ton aus Bentonit und Montmorillonit-Tonen ausgewählt wird.

**16.** Waschmittelzusammensetzung nach Anspruch 14, dadurch gekennzeichnet, daß dieser Ton in Pulverform in einer Menge von 0,5 bis 10% bezogen auf das Gewicht der Zusammensetzung vorhanden ist.

**17.** Waschmittelzusammensetzung nach Anspruch 16, dadurch gekennzeichnet, daß dieser Ton in Pulverform in einer Menge von 0,5 bis 1,5 Gew.-% der Zusammensetzung vorhanden ist.

**18.** Waschmittelzusammensetzung nach Anspruch 16, dadurch gekennzeichnet, daß dieser Ton in Pulverform in einer Menge von 1,5 bis 10 Gew.-% der Zusammensetzung vorhanden ist.

**19.** Waschmittelzusammensetzung nach Anspruch 1, dadurch gekennzeichnet, daß das synthetische Öl ausgewählt wird aus Isopropylmyristat, -palmitat und -stearat, 2-Cetyldodecanol, Decyloleat, Derivaten von Isostearinsäure, Diisopropyladipat und Myristyllactat.

**20.** Verfahren zum Reinigen von Haut und Haaren, dadurch gekennzeichnet, daß eine Zusammensetzung nach einem der vorhergehenden Ansprüche mit Wasser vermischt wird, um eine Emulsion zu bilden, und auf die Haut oder die Haare aufgetragen wird, wobei die Emulsion dann durch Spülen entfernt wird.

**21.** Verwendung eines Saccharose-Esters, ausgewählt aus Saccharose-mono- und -dilaurat, -mono- und -dipalmitat und -mono- und -distearat, beim Herstellen einer Waschmittelzusammensetzung, die zum Reinigen von Haut und Haaren brauchbar ist, die eine Ölkomponente, ausgewählt aus tierischen, pflanzlichen, Mineral- und synthetischen Ölen sowie Mischungen dieser, und eine emulgierende Mischung umfaßt, welche mindestens zwei Emulgiermittel, ausgewählt aus anionischen, kationischen, nicht-ionischen und amphoteren Emulgiermitteln, umfaßt.

**Revendications**

**1.** Composition détergente contenant une huile choisie parmi les huiles animales, végétales, minérales et synthétiques, ainsi que leurs mélanges, et un mélange émulsifiant comprenant au moins deux agents émulsifiants, choisis parmi les agents émulsifiants anioniques, cationiques, non-ioniques et amphotères, caractérisée par le fait que ledit mélange émulsifiant comprend, en tant que l'un des au moins deux agents émulsifiants, un ester de saccharose choisi parmi les mono- et di-laurates, mono- et di-palmitates, et mono- et di-stéarates de saccharose.

**2.** Composition détergente selon la revendication 1, caractérisée par le fait que ledit ester de saccharose est présent dans une quantité comprise entre 0,05 et 2% en poids par rapport au poids de la composition.

**3.** Composition détergente selon la revendication 1, caractérisée par le fait que ladite huile ou ledit mélange d'huiles est au moins partiellement remplacé(e) par de la gélatine végétale.

**4.** Composition détergente selon la revendication 1, caractérisée par le fait que la teneur de ladite huile ou dudit mélange d'huile est comprise entre 80 et 98% en poids de la composition.

**5.** Composition détergente selon la revendication 1, caractérisée par le fait que ledit mélange émulsifiant comprend un agent émulsifiant choisi parmi :
   - les sulfates d'alcools gras et dérivés
   - les dérivés d'acides aminés et de bétaïne ;
   - les acides et alcools gras condensés avec l'oxyde d'éthylène ;
   - les dérivés de polyéthylène et de polyoxypropylène.

10

**6.** Composition détergente selon la revendication 1, caractérisée par le fait que la teneur dudit mélange émulsifiant est comprise entre 2 et 20% en poids de la composition.

**7.** Composition détergente selon la revendication 1, caractérisée par le fait que ladite huile minérale est choisie parmi l'huile de vaseline et l'huile de silicone.

**8.** Composition détergente selon la revendication 1, caractérisée par le fait que ladite huile végétale est choisie parmi l'huile d'amande, l'huile de germe de maïs, l'huile d'olive, l'huile de noix, l'huile de soja, l'huile de pépins de raisin, l'huile de sésame, l'huile d'arachide, l'huile de copra, l'huile d'avocat, l'huile de tournesol et l'huile de maïs.

**9.** Composition détergente selon la revendication 1, caractérisée par le fait que ladite huile animale est choisie parmi l'huile de tortue, l'huile de vison, l'huile de marmotte et l'huile de lanoline.

**10.** Composition détergente selon la revendication 1, caractérisée par le fait qu'elle contient en outre un agent hydratant.

**11.** Composition détergente selon la revendication 10, caractérisée par le fait que ledit agent hydratant est choisi le glycérol, l'urée et les acides aminés non-dispersibles et non ou médiocrement solubles dans l'eau, à la fois seuls et en combinaison.

**12.** Composition détergente selon la revendication 11, caractérisée par le fait que lesdits acides aminés sont choisis parmi la valine, l'isovaline, la leucine et l'isoleucine.

**13.** Composition détergente selon la revendication 1, caractérisée par le fait qu'elle contient une vitamine choisie parmi la vitamine A, E et F.

**14.** Composition détergente selon la revendication 1, caractérisée par le fait qu'elle contient en outre de l'argile sous la forme pulvérulente.

**15.** Composition détergente selon la revendication 14, caractérisée par le fait que ladite argile est choisie parmi les argiles de type bentonite et montmorillonite.

**16.** Composition détergente selon la revendication 14, caractérisée par le fait que ladite argile pulvérulente est présente dans une quantité de 0,5 à 10% par rapport au poids de la composition.

**17.** Composition détergente selon la revendication 16, caractérisée par le fait que ladite argile pulvérulente est présente dans la quantité de 0,5 à 1,5% en poids de la composition.

**18.** Composition détergente selon la revendication 16, caractérisée par le fait que ladite argile pulvérulente est présente dans la quantité de 1,5 à 10% en poids de la composition.

**19.** Composition détergente selon la revendication 1, caractérisée par le fait que ladite huile synthétique est choisie parmi les myristate, palmitate et stéarate d'isopropyle, le cétyl dodécanol, l'oléate de décyle, les dérivés de i'acide isostéarique, l'adipate de diisopropyle et le lactate de myristyle.

**20.** Procédé pour le nettoyage de la peau et des cheveux, caractérisé par le fait qu'une composition telle que défini à l'une des revendications précédentes est mélangée avec de l'eau pour former une émulsion et est appliquée sur la peau ou les cheveux, l'émulsion étant ensuite éliminée par rinçage.

**21.** Utilisation d'un ester de saccharose choisi parmi les mono- et di-laurates, mono- et di-palmitates, et mono- et di-stéarates de saccharose dans la fabrication d'une composition détergente utile pour le nettoyage de la peau et des cheveux, comprenant un composant de type huile choisi parmi les huiles animales, végétales, minérales et synthétiques, ainsi que leurs mélanges, et un mélange émulsifiant comprenant au moins deux agents émulsifiants choisis parmi les agents émulsifiants anioniques, cationiques, non-ioniques et amphotères.